(19) Europäisches Patentamt / European Patent Office / Office européen des brevets

(11) EP 4 736 833 A1

(12) **EUROPEAN PATENT APPLICATION**

(43) Date of publication:
**06.05.2026 Bulletin 2026/19**

(21) Application number: **24210551.8**

(22) Date of filing: **04.11.2024**

(51) International Patent Classification (IPC):
***A61K 6/16*** *(2020.01)* ***A61K 6/62*** *(2020.01)*
***A61K 6/77*** *(2020.01)* ***A61K 6/836*** *(2020.01)*
***A61K 6/887*** *(2020.01)*

(52) Cooperative Patent Classification (CPC):
(C-Sets available)
**A61K 6/887; A61K 6/16; A61K 6/62; A61K 6/77; A61K 6/836** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **DENTSPLY SIRONA Inc.**
  **York, PA 17401 (US)**
- **Dentsply DeTrey GmbH**
  **78467 Konstanz (DE)**

(72) Inventors:
- **Trötschler, Tobias**
  **78467 Konstanz (DE)**
- **Qizhou, Dai**
  **78467 Konstanz (DE)**
- **Fredrich, Sebastian**
  **78467 Konstanz (DE)**

(74) Representative: **Crow, Martin**
**Venner Shipley LLP**
**200 Aldersgate**
**London EC1A 4HD (GB)**

(54) **POLYMERIZABLE DENTAL COMPOSITION**

(57) The present invention relates to a polymerizable dental composition comprising a specific monomeric system containing at least a specific high viscosity performer monomer and a specific low viscosity thinner monomer with similar refractive indices. The present invention also relates to the use of the specific monomeric system for the preparation of a crosslinked dental composition. Finally, the present invention relates to the use of a specific compound for stabilizing a polymerized dental composition.

EP 4 736 833 A1

(52) Cooperative Patent Classification (CPC): (Cont.)

C-Sets
**A61K 6/887, C08L 47/00**

## Description

### Field of the Invention

The present invention relates to a polymerizable dental composition comprising a specific monomeric system containing at least a specific high viscosity performer monomer and a specific low viscosity thinner monomer with similar refractive indices. The present invention also relates to the use of the specific monomeric system for the preparation of a crosslinked dental composition. Finally, the present invention relates to the use of a specific compound for stabilizing a polymerized dental composition.

### Background of the Invention

Polymerizable dental compositions including dental restorative materials are known. Generic compositions mainly contain polymerizable monomers, an initiator system, and further components such as particulate fillers, solvents and UV-stabilizer depending on the clinical use.

Polymerizable monomers in dental restorative materials such as in dental composites must meet several requirements. In addition to the mechanical and rheological properties, also the optical properties must be taken into account. Simultaneously optimizing all these properties for a dental material is a major challenge. Any trade-off can result in poor performance of the material. For example, high viscosity monomers and a high filler content are required for high mechanical strength and stability. On the other hand, the viscosity of the resin matrix needs to be low for achieving good handling performance while keeping the filler content high. Accordingly, a thinner or reactive diluent may be used for reducing the viscosity of the dental restorative material. A combination of high viscosity monomers as performers and a thinner allows to adjust mechanical properties and viscosity for dental materials. However, it is difficult to adjust the rheological properties of the monomer mixture while not changing the optical properties at the same time.

Thinners or reactive diluents are low-viscosity, reactive monomers or oligomers added to a polymerizable dental composition to reduce its viscosity while still being capable of chemically reacting with other components in the composition during the polymerization process. For example, ethylene glycol dimethacrylate (EGDMA) or triethylene glycol dimethacrylate (TEGDMA) are commonly used as reactive diluents in polymerizable dental compositions. However, EGDMA or TEGDMA reduce the refractive index of the polymerized dental resin matrix given that, for example, the refractive index of polymerized TEGDMA is typically only around 1.46 to 1.48 which is significantly different the refractive index of a dental filler material.

The refractive index is an important property for dental materials in that the refractive index of the polymerizable liquid should match the dental filler for ensuring that the optical properties of dental composites match those of natural tooth structures.

US 10,849,724 discloses in Example 2 a specific urethane monomer (UCDPMAA) having desirable properties as a performer. UCDPMAA is a high performing monomer yielding good mechanical performance after polymerization. However, UCDPMAA has a high viscosity with the tendency to crystallize, which is not suitable in neat form for dental materials.

### Summary of the Invention

According to a first aspect, it is the problem of the present invention to provide a filler-containing polymerizable dental composition having adjustable viscosity of the liquid and an essentially constant refractive index matching the refractive index of a particulate filler such as a dental glass after polymerization, and providing a natural tooth-like opacity after curing, good handling properties, sufficient mechanical stability, easy incorporation of further functionality without disturbing the esthetics, good biocompatibility and low risk of health issues. Moreover, it is the problem of the present invention to provide a use of the specific monomeric system for the preparation of a crosslinked dental composition.

The problem according to the first aspect is solved by a polymerizable dental composition comprising

(a) 10 to 99 percent by weight based on the total weight of the dental composition of a homogenous mixture of polymerizable monomers, which comprises

(a1) 5 to 99.5 percent by weight based on the total weight of the mixture of a first polymerizable component comprising one or more thinner compounds of the following formula (I):

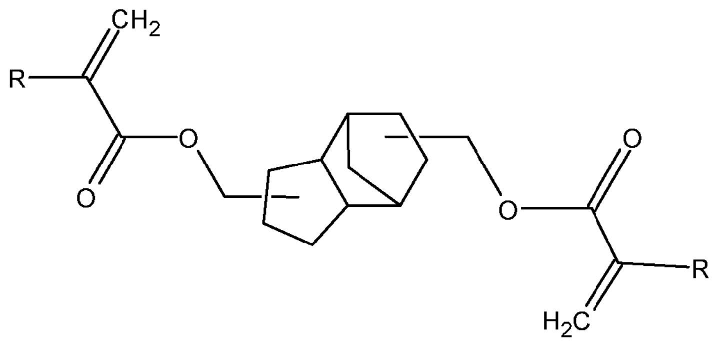

(I)

wherein the R, which may be the same or different, independently from each other represent a hydrogen atom or a $C_{1-4}$ alkyl group which may be substituted by one or more fluorine atoms; and
(a2) 0.5 to 90 percent by weight based on the total weight of the mixture of one or more performer compounds of the following formula (II):

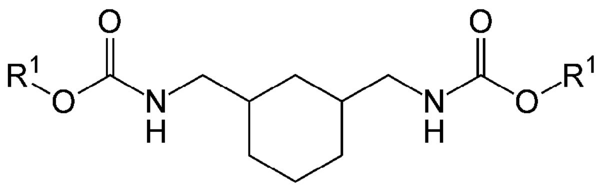

(II)

wherein the $R^1$ which may be the same or different, independently from each other is selected from a group of the following formulas (II-a) or (II-b):

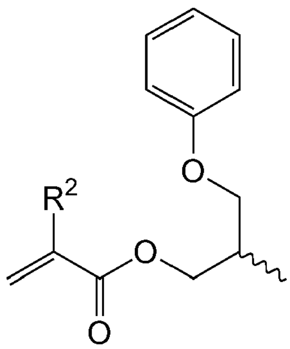

(II-a)

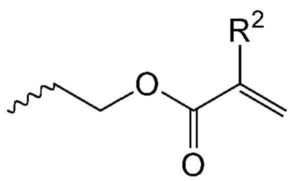

(II-b)

wherein $R^2$ represents a hydrogen atom or a $C_{1-4}$ alkyl group which may be substituted by one or more fluorine atoms; and
(a3) optionally a third polymerizable component;

(b) a polymerization initiator system, and
(c) 10 to 90 percent by weight of a particulate filler.

According to a second aspect, it is the problem of the invention to provide a polymerizable dental composition having improved natural appearance when polymerized and enhanced UV resistance

The problem according to the second aspect is solved by a compound of the following formula (III):

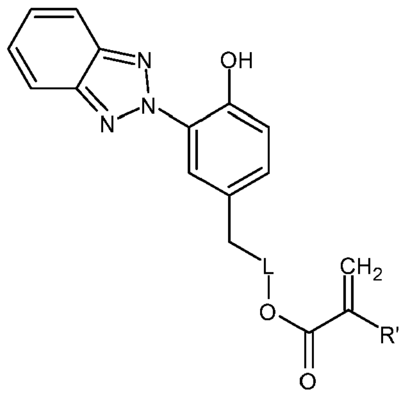

(III)

wherein

R' is a hydrogen atom or a $C_{1-4}$ alkyl group, and
L is a divalent linker;

for use in stabilizing a polymerized dental composition, notably a polymerized polymerizable dental composition according to the first aspect.

The present invention is based on the recognition that thinners used in the prior art have a low refractive index and/or diminish the mechanical performance. Tricyclodecane dimethanol diacrylate as particular example of the compound of formula (I) has a high refractive index of around 1.505, a low viscosity of about 0.2 Pa s and does not impair the mechanical performance of the resulting composite formulation.

The performer mixture (a2) according to the present invention containing a compound of formula (II) has a relatively high refractive index of around 1.510 and is a high performing monomer yielding good mechanical performance after polymerization. However, (a2) has a high viscosity of about 630 Pa s, with the tendency to crystallize, which is not suitable in neat form for dental materials. With combination of (a1) and (a2), it is possible to create monomer mixtures with a widely selectable viscosity from 0.5 Pa s to 600 Pa s but in a narrow range of the refractive index from 1.505 to 1.510, without deteriorating the high mechanical properties of (a2).

In case of the optical performance, the refractive index of the liquid and the particulate filler such as a dental glass are crucial. The refractive index of the monomers changes to a higher value due to the shrinkage during the polymerization process. Therefore, the refractive index of 1.505 to 1.510 of the monomer mixture increases to around 1.53 after polymerization, which is a typical refractive index for, for example, dental glasses. A similar refractive index between the polymer and the glass generates low opacity and thus an optically high-quality dental material. Furthermore, with similar refractive indices and large differences in viscosity of (a1) and (a2), dental materials with a great variety of viscosities can be prepared, keeping with high optical and high mechanical performance. This allows preparing a desirable combinations of properties with little trade-off.

If most of the monomer mixture consists of monomers (a1) and (a2), the refractive index and thus the optical features of the composite remain robust and stable even if small amounts of other monomers (a3) are added. Especially functional monomers containing among others initiating, inhibiting, or UV-stabilizing features, activate their effect on initiation, inhibition, UV-stabilization, etc. already with addition of small quantities.

On the contrary, addition of larger amounts of additional monomer to (a1) and (a2) can fine-tune the opacity on the final composite to achieve superior natural esthetics.

**Brief description of the Figures**

Figure 1 shows the UV-stability of composites with (2-(2-Hydroxy-5-methylphenyl)benzotriazole) or (2-[3-(2H-Benzotriazol-2-yl)-4- hydroxyphenyl]ethyl methacrylate)).

Figure 2 shows leaching of polymerized plates in acetonitrile at room temperature for 72 h containing (2-(2-Hydroxy-5-methylphenyl)benzotriazole) or (2-[3-(2H-Benzotriazol-2-yl)-4-hydroxyphenyl]ethyl methacrylate)).

Figure 3 shows the refractive index of the liquid formulation and the opacity of the cured composite for each formulation

1 to 14.

## Detailed description of the Preferred Embodiments

The terms "polymerization" or "polymerizable" relate to the combining by covalent bonding of many smaller molecules, such as monomers in the form of radical-polymerizable or cationically polymerizable compounds, to form larger molecules, that is, macromolecules or polymers. The monomers may be combined to form only linear macromolecules or they may be combined to form three-dimensional macromolecule, commonly referred to as crosslinked polymers. In case of a higher conversion rate of the polymerizable monomer, the amount of multifunctional monomers may be reduced or the leaching problem may be alleviated.

The term "photocurable" refers to a dental composition that will radically and/or cationically polymerize into a crosslinked polymer network when irradiated for example with actinic radiation such as ultraviolet (UV), visible, or infrared radiation. "Actinic radiation" is any electromagnetic radiation that is capable of producing photochemical action and can have a wavelength of at least 150 nm and up to and including 1250 nm, and typically at least 300 nm and up to and including 750 nm.

The term "radical-polymerizable" as used herein in connection with compounds (a) means any compound capable of radical polymerization. Typically, compounds (a) are radical-polymerizable due to a polymerizable double bond, preferably one or more carbon-carbon double bonds. Examples of the polymerizable double bond include vinyl, conjugated vinyl, allyl, acryl, methacryl and styryl. More preferably, the polymerizable double bond is selected from the group consisting of acryl, methacryl and styryl. Acryl and methacryl may be (meth)acryloyl or (meth)acrylamide. Most preferably, for compounds (a), the polymerizable double bound is acryl or methacryl and/or acrylamide or methacrylamide.

The term "cationically polymerizable" as used herein in connection with compounds (a) means any compound capable of cationic polymerization. Typically, compounds (a) are cationically polymerizable due to the presence of a functional group having a carbon-carbon double bond such as a vinyl ether group.

The term "polymerization initiator system" refers to a system comprising (i) 0.001 to 10 weight%, preferably 0.01 to 7 weight%, particularly 0.05 to 6 weight%, especially 0.05 to 5 weight% at least one photoinitiator based on the total weight of the dental composition and (ii) 0.001 to 10 weight%, preferably 0.01 to 7 weight%, particularly 0.05 to 6 weight%, especially 0.05 to 5 weight% based on the total weight of the dental composition at least one co-initiator. Optionally, the polymerization initiator system may further comprise (iii) an additional electron-donor.

The term "photoinitiator" used in connection with the compound (i) refers to a compound that is activated, for example by forming free radicals, typically by exposure to light or interaction with another compound such as co-initiator compound (ii) and/or electron-donor (iii) in a photochemical process. Compounds (i), (ii), and (iii) may be substituted with a polymerizable group, preferably with an acrylate- or methacrylate group for reducing the leaching from the polymerized dental composition.

The present invention relates to a polymerizable, notably a photocurable dental composition. The photocurable dental composition may be a dental composite, a resin-modified glass ionomer cement (RMGIC), a provisional crown and bridge material, a lab composite, a pit and fissure sealer, a dental impression material, and a dental adhesive.

The polymerizable dental composition comprises 10 to 99 percent by weight based on the total weight of the dental composition of a homogenous mixture of polymerizable monomers as component (a). According to a preferred embodiment, the polymerizable dental composition comprises 20 to 50 percent by weight based on the total weight of the dental composition of the homogenous mixture of polymerizable monomers of component (a). Still more preferably, the homogenous mixture of polymerizable monomers of component (a) is contained in the polymerizable dental composition in an amount of from 25 to 40 percent by weight based on the total weight of the dental composition.

Component (a) comprises 5 to 99.5 percent by weight based on the total weight of the mixture of a first polymerizable component comprising one or more specific compounds as component (a1) and 0.5 to 90 percent by weight based on the total weight of the dental composition of a second polymerizable compound as component (a2).

According to a preferred embodiment, the first polymerizable component comprises at least two compounds of formula (I), preferably wherein the two or more compounds are enantiomers, diastereomers and constitutional isomers.

The one or more specific compounds of component (a1) are compounds of the following formula (I):

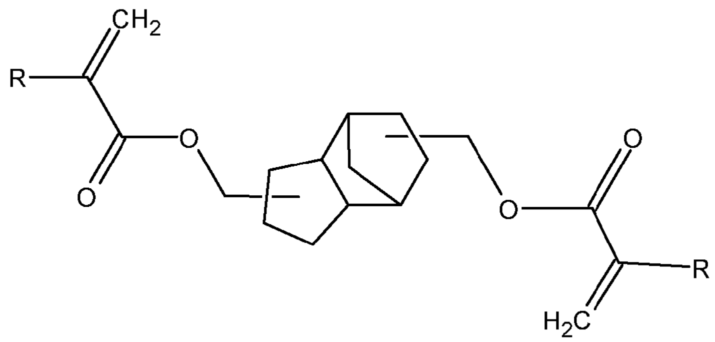

(I)

In a compound of formula (I), the R may be the same or different. Preferably, the R are the same. The R independently from each other represent a hydrogen atom or a $C_{1-4}$ alkyl group. Preferably, the R represent a hydrogen atom or a methyl group. When R is a $C_{1-4}$ alkyl group, the $C_{1-4}$ alkyl group may be substituted by one or more fluorine atoms.

Preferably, the one or more compounds of formula (I) comprise or consist of compounds of the following formula (I-a) and/or (I-b):

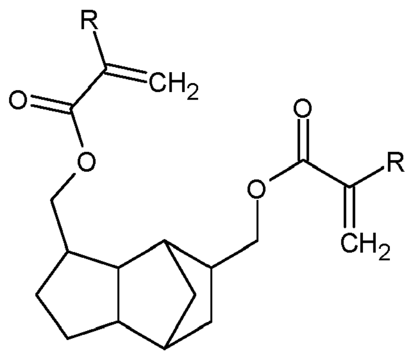

(I-a)

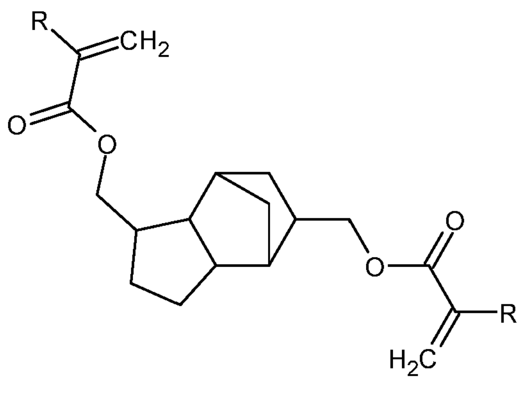

(I-b)

In formula (I-a) and (I-b), the R are as defined above. According to a preferred embodiment, the compounds of formula (I-a) and (I-b) are tricyclodecane dimethanol diacrylate compounds wherein R represents a hydrogen atom.

Preferably, component (a1) is contained in 10 to 85 percent by weight, more preferably 20 to 80 percent by weight, especially 25 to 75 percent by weight based on the total weight of the polymerizable mixture.

The one or more specific compounds of component (a2) are compounds of the formula (II):

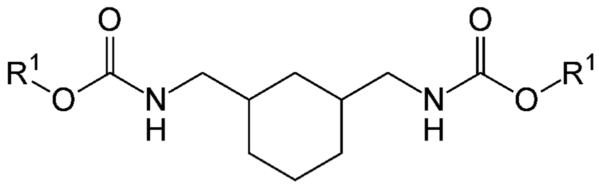

(II)

wherein $R^1$ may be the same or different, independently from each other represent one of the moieties of the following formulars:

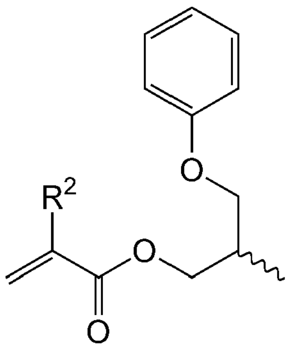

(II-a)

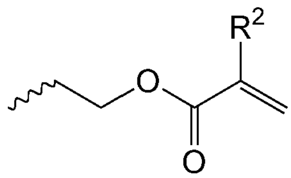

(II-b)

wherein $R^2$ may be the same or different independently from each other represent a hydrogen atom or a $C_{1-4}$ alkyl group which may be substituted by one or more fluorine atoms.

Preferably, one or more compounds of formula (II) comprise or consist of compounds of the following formula (II-1) and/or (II-2) and/or (II-3):

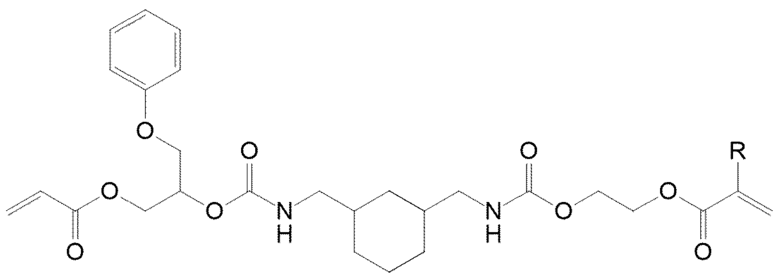

(II-1)

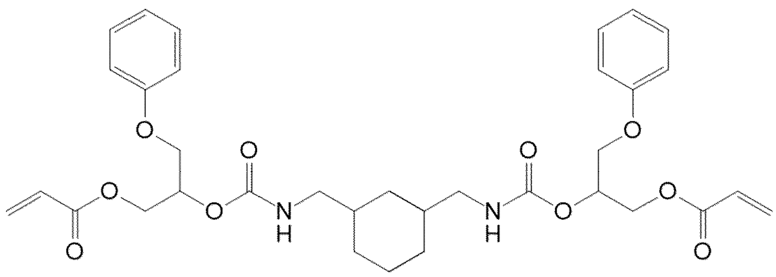

(II-2)

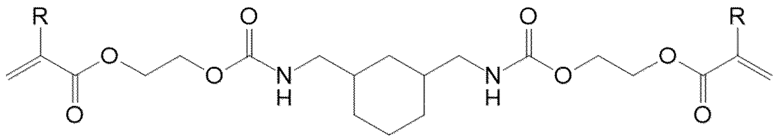

(II-3)

Tricyclodecane dimethanol diacrylate has a dynamic viscosity in the range of 0.100 to 0.250 Pa s, notably about 0.187 Pa s.

Preferably, component (a2) is contained in 10 to 85 percent by weight, particularly 20 to 80 percent by weight, especially 25 to 75 percent by weight based on the total weight of the mixture.

The third polymerizable component (a3) may comprise one or more polymerizable compounds which may be radical-polymerizable or cationically polymerizable. The one or more radical-polymerizable compounds (a3) may preferably be radical-polymerizable N-substituted alkyl acrylic or acrylic acid amide monomers or a (meth)acrylate compounds.

A radical-polymerizable N-substituted alkyl acrylic or acrylic acid amide monomer may be preferably selected from compounds characterized by one of the following formulae (X), (XI) and (XII):

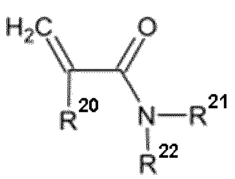

(X)

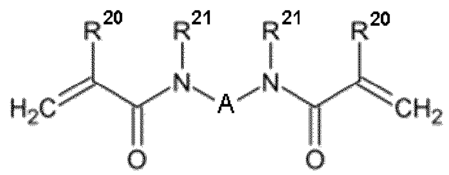

(XI)

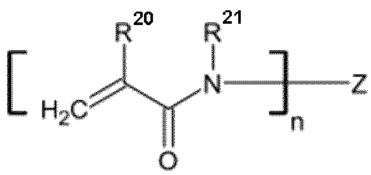

(XII).

In formulae (X), (XI) and (XII), $R^{20}$, $R^{21}$ and $R^{22}$ independently represent a hydrogen atom or a C1 to C8 alkyl group; A represents a divalent substituted or unsubstituted organic residue having from 1 to 10 carbon atoms, whereby said organic residue may contain from 1 to 3 oxygen and/or nitrogen atoms; Z represents a saturated at least trivalent substituted or unsubstituted $C_{1-8}$ hydrocarbon group, a saturated at least trivalent substituted or unsubstituted cyclic $C_{3-8}$ hydrocarbon group, and n is at least 3.

Preferably, the one or more radical-polymerizable compounds (a3) include bisacrylamides such as N,N'-diethyl-1,3-bisacrylamido-propan (BADEP), 1,3-bisacrylamido-propan (BAP), 1,3-bisacrylamido-2-ethyl-propan (BAPEN), N,N'-(2E)-2-butene-1,4-diylbis[N-2-propen-1-yl-2-propenamide] (BAABE) , N,N-di(cyclopropyl acrylamido) propane (BCPBAP) and N,N'-(2-hydroxy-1,3-propanediyl)bis[N-2-propen-1-yl-2-propenamide] (DAAHP).

Alternatively or additionally, for the one or more radical-polymerizable compounds (a3), a (meth)acrylate compound may be selected from the group of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bisGMA"), glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl

carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl) propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of radical-polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Preferably, the one or more radical-polymerizable compounds (a3) contain one or two radical-polymerizable groups, more preferably two radical-polymerizable groups, such as acrylamides or bisacrylamides like Bis-GMA, TEGDMA, UDMA, BADEP, BAP, BAPEN, BAABE, BCPBAP and 9,9-Bis[4-(2-acryloyloxyethoxy)phenyl]fluorene (BFDA).

According to a specific embodiment, the third polymerizable component (a3) comprises one or more polymerizable monomers selected from bisphenol A-glycidyl methacrylate (BisGMA), urethane dimethacrylate (UDMA), and ethoxylated bisphenol A dimethacrylate (BisEMA), polycarbonate urethane dimethacrylate (PCUDMA).

The third polymerizable component (a3) may be contained in an amount of 0.001 to 90 percent by weight, preferably 0.01 to 70 percent by weight, more preferably 0.01 to 60 percent by weight, especially 0.01 to 40 percent by weight based on the total weight of the polymerizable mixture. According to a specific embodiment, the third polymerizable component (a3) is contained in an amount of at most 40 percent by weight, more preferably at most 30 percent by weight based on the total weight of the polymerizable mixture, still more preferably at most 25 percent by weight based on the total weight of the polymerizable mixture.

Preferably, the refractive index of the third polymerizable component (a3) is in the range of 1.49 to 1.62.

According to a preferred embodiment, the ratio of the refractive index of component (a1) and the refractive index of component (a2) $n_{(a1)} / n_{(a2)}$ is in the range of 0.95 to 1.05, more preferably 0.97 to 1.03.

A polymerizable dental composition further comprises a polymerization initiator system as component (b). The polymerization initiator system is preferably soluble in the mixture. Specifically, the polymerization initiator may preferably be fully dissolved in the required amount in the polymerizable dental composition. Typically, the polymerizable dental composition contains 0.01 to 20 percent by weight, more preferably 0.1 to 10 percent by weight based on the total amount of the composition of the polymerization initiator system of component (b).

As a polymerization initiator system according to component (b), any compound or system, capable of initiating the copolymerization reaction according to the present invention may be suitably used. The polymerization initiator according to component (b) may be a photoinitiator or a redox initiator or a mixture thereof.

A suitable photoinitiator compound is a compound having a light absorption maximum in the range from 300 to 800 nm, in particular 350 to 500 nm. The polymerization initiator system (b) may comprise one or a mixture of two or more photoinitiator compound(s). The photoinitiator compound may be an acylphosphine oxide or a 1,2-diketone. Suitable 1,2-diketone photoinitiator compounds may be selected from the group consisting of camphorquinone, benzil, 2,2'-3 3'- and 4,4'-dihydroxylbenzil, 2,3-butanedione, 2,3-pentanedione, 2,3-hexanedione, 3,4-hexanedione, 2,3-heptanedione, 3,4-heptanedione, 2,3-octanedione, 4,5-octanedionefuril, 1,2-cyclohexanedione, 1,2-naphthaquinone, and acenaphthaquinone. Camphorquinone is preferred.

Suitable electron donor compounds include substituted amines, e.g. ethyl dimethylaminobenzoate, dibenzylphenylamine, Troeger's base. shoOther suitable tertiary amine reducing agents may be selected from aromatic tertiary amine include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, 4-N,N-dimethylaminobenzoic acid ethyl ester, 4-N,N-dimethylaminobenzoic acid methyl ester, 4-N,N-dimethylaminobenzoic acid n-butoxyethyl ester, 4-N,N-dimethylaminobenzoic acid 2-(methacryloyloxy) ethyl ester, 4-N,N-dimethylaminobenzophenone ethyl 4-(N,N-dimethylamino)benzoate and N,N-dimethylaminoethyl methacrylate. Examples of an aliphatic tertiary amine include trimethylamine, triethylamine, N-methyldiethanolamine, N-ethyldiethanolamine, N-n-butyldiethanolamine, N-lauryldiethanolamine, triethanolamine, 2-(dimethylamino) ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

The amine reducing agent may be present in the composition in an amount from 0.01 percent by weight to 2.0 percent by weight, based on the total weight of the composition.

Suitable photoinitiators may also include phosphine oxides typically having a functional wavelength range of about 380 nm to about 1200 nm. Examples of phosphine oxide free radical initiators with a functional wavelength range of about 380 nm to about 450 nm include acyl and bisacyl phosphine oxides such as those described in US 4,298,738, US 4,324,744 US and 4,385,109 and EP 0 173 567. Specific examples of the acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphonate, and 2,4,6-trimethylbenzoylethoxyphenylphosphine oxide. Commercially available phosphine oxide photoinitiators capable of free-radical initiation when irradiated at wavelength ranges of greater than about 380 nm to about 450 nm include bis(2,4,6-trimethylbenzoyl)phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide initiator is present in the composition in catalytically effective amounts, such as from 0.1 percent by weight to 1.0 percent by weight, based on the total weight of the composition.

A suitable redox initiator comprises a reducing and an oxidizing agent, which typically react with or otherwise cooperate with one another to produce free-radicals capable of initiating polymerization of polymerizable double bonds in component (a) in a dark reaction, independent from the presence of light. The reducing and oxidizing agents are selected so that the polymerization initiator system is sufficiently storage-stable and free of undesirable colorization to permit storage and use under typical dental conditions. Moreover, the reducing and oxidizing agents are selected so that the polymerization initiator system is sufficiently miscible with the resin system to permit dissolution of the polymerization initiator system in the composition.

Useful reducing agents include ascorbic acid, ascorbic acid derivatives, and metal complexed ascorbic acid compounds as described in US 5,501,727; amines, namely tertiary amines, such as 4-tert-butyl dimethylaniline; aromatic sulfinic salts, such as p-toluenesulfinic salts and benzenesulfinic salts; thioureas, such as 1-ethyl-2-thiourea, tetraethyl thiourea, tetramethyl thiourea, 1,1-dibutyl thiourea, and 1,3-dibutyl thiourea; and mixtures thereof. Other secondary reducing agents may include cobalt (II) chloride, ferrous chloride, ferrous sulfate, hydrazine, hydroxylamine, salts of a dithionite or sulfite anion, and mixtures thereof.

The reducing agent may be present in the composition in an amount from 0.1 percent by weight to 5.0 percent by weight, based on the total weight of the composition.

Suitable oxidizing agents include persulfuric acid and salts thereof, such as ammonium, sodium, potassium, caesium, and alkyl ammonium salts. Additional oxidizing agents include peroxides such as benzoyl peroxides, hydroperoxides such as cumyl hydroperoxide, t-butyl hydroperoxide, and amyl hydroperoxide, as well as salts of transition metals such as cobalt (III) chloride and ferric chloride, cerium (IV) sulfate, perboric acid and salts thereof, permanganic acid and salts thereof, perphosphoric acid and salts thereof, and mixtures thereof. One or more different oxidizing agents or one or more different reducing agent may be used in the polymerization initiator system. Small quantities of transition metal compounds may also be added to accelerate the rate of redox cure. The reducing and oxidizing agents are present in amounts sufficient to permit an adequate free-radical reaction rate.

The oxidizing agent may be present in the composition in an amount from 0.1 percent by weight to 5.0 percent by weight, based on the total weight of the composition.

The reducing or oxidizing agents may be microencapsulated for enhancing shelf stability of the composition, and if necessary permitting packaging the reducing and oxidizing agents together (US 5,154,762). Appropriate selection of an encapsulant may allow combination of the oxidizing and reducing agents and even of an acidfunctional component and optional filler in a storage-stable state. Moreover, appropriate selection of a water-insoluble encapsulant allows combination of the reducing and oxidizing agents with the particulate reactive glass and water in a storage-stable state.

Preferably, the polymerizable dental composition contains 0.001 to 10 weight%, more preferably 0.01 to 7 weight%, particularly 0.05 to 6 weight%, especially 0.05 to 5 weight% of a polymerization initiator system (b) based on the total weight of the dental composition.

A polymerizable dental composition further comprises 10 to 90 percent by weight of a particulate filler as component (c). According to a preferred embodiment, the polymerizable dental composition comprises the particulate filler in an amount of from 40 to 90 percent by weight, more preferably 45 to 85 percent by weight, based on the total weight of the composition.

Suitable particulate fillers may be selected from fillers currently used in dental compositions. The filler should be finely divided and preferably has a maximum particle diameter less than about 100 μm and an average particle diameter

less than about 10 μm. The filler may have a unimodal or polymodal (e.g., bimodal) particle size distribution.

The filler can be an inorganic material. It can also be a crosslinked organic material that is insoluble in the one or more radical-polymerizable compounds (a), and is optionally filled with inorganic filler. The filler can be radioopaque. Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. According to a preferred embodiment, the polymerizable composition according to the present invention, particulate filler comprises a glass.

Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler may also be a filler obtainable by a process for the preparation of composite filler particles, comprising:

a) coating a particulate filler having a median particle size (D50) of from 1 to 1200 nm with a coating composition containing a film-forming agent forming a coating layer on the surface of the particulate filler, said coating layer displaying reactive groups on the surface of the coating layer, said reactive groups being selected from addition polymerizable groups and step-growth polymerizable groups, thereby forming a coated particulate filler; subsequently or concurrently

b) agglomerating the coated particulate filler, optionally in the presence of a further crosslinking agent and optionally in the presence of a further particulate filler not displaying reactive groups, for providing a granulation of the coated particulate filler wherein the granulation contains the coated particulate filler particles and the optional further particulate filler particles separated from and connected to each other by at least one coating layer, whereby the at least one coating layer may be crosslinked by crosslinking groups obtained by reacting the reactive groups and optionally a further crosslinking agent;

c) optionally milling, classifying and/or sieving the granulation of the coated particulate filler; and

d) optionally further crosslinking the granulation of the coated particulate filler; for providing composite filler particles having a median particle size (D50) of from 1 to 70 μm, wherein reactive groups are transformed into crosslinking groups obtained by reacting reactive groups and optionally a further crosslinking agent, and wherein the particulate filler is the main component by volume of the composite filler particles as further described in EP-A 2 604 247.

The polymerizable dental compositions of the present invention may further contain one or more UV-stabilizers, solvents, polymerization inhibitors, pigments, a rheological modifier, a fluorescent agent, an acidic component, an X-ray opaquer, a nonreactive diluent, coupling agents to enhance reactivity of fillers, rheology modifiers, and surfactants.

A UV-stabilizer may be selected from a compound of the following formula (III):

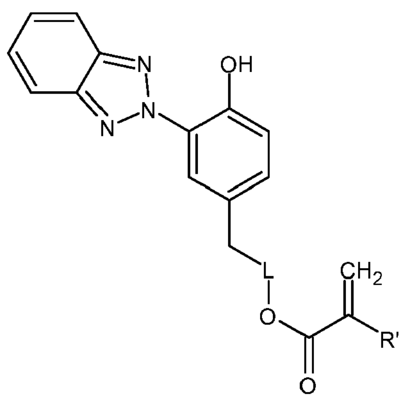

(III)

wherein

R' is a hydrogen atom or a $C_{1-4}$ alkyl group, and

L is a single bond or a divalent linker.

In a compound of formula (III), R' is preferably a hydrogen atom or a methyl group.

In a compound of formula (III), L is single bond or a divalent linker. When L is a single bond, then the ester group is attached via a methylene group to the aromatic ring. The divalent linker may be a straight-chain, branched or cyclic linker.

Preferably, the linker is a straight-chain hydrocarbon linker. More preferably, L is a straight chain $C_{1-18}$ alkylene group which may contain one or more heteroatoms selected from oxygen atoms and sulfur atoms. According to a preferred embodiment, L is a methylene group. According to a specific embodiment, L is an alkylene group or a polyoxyalkylene group.

The UV-stabilizer including a compound of formula (III), may be contained in the dental composition in an amount of 0.001 to 10 percent by weight, preferably 0.01 to 7 percent by weight, particularly 0.05 to 6 percent by weight, especially 0.05 to 5 percent by weight based on the total weight of the dental composition. The specific UV-stabilizer is integrated into the polymer network and does not tend to leach from the polymerized dental composition.

An optional solvent may be selected from water, alcohols such as methanol, ethanol, propanol (n-, i-), butanol (n-, iso-, tert.-), ketones such as acetone or the like. The polymerizable dental composition of the present invention may preferably comprise 10 to 75 percent by weight based on the total weight of the composition of a solvent. However, according to a general embodiment, the polymerizable dental composition does not contain a solvent.

A polymerization inhibitor may be selected from the group comprising butylated hydroxytoluene (BHT), hydroquinone (HQ), methyl ether of hydroquinone (MeHQ), benzoquinone and tert-butyl catechol.

The polymerization inhibitor may be contained in an amount of from 0.0001 to 10 percent by weight, preferably 0.001 to percent by weight, particularly 0.005 to 6 percent by weight, especially 0.005 to 5 percent by weight based on the total weight of the dental composition.

Pigments may be contained in an amount of from 0.0001 to 1 percent by weight based on the total weight of the dental composition.

A polymerizable dental composition according to the present invention may preferably be selected from a dental composite, a resin-modified glass ionomer cement (RMGIC), a provisional crown and bridge material, a lab composite, a pit and fissure sealer, a dental impression material, and a dental adhesive.

According to a preferred embodiment, the polymerizable dental composition is extruded from a dental container, preferably a compule.

According to the second aspect of the present invention, the present invention provides a use of a compound of the following formula (III):

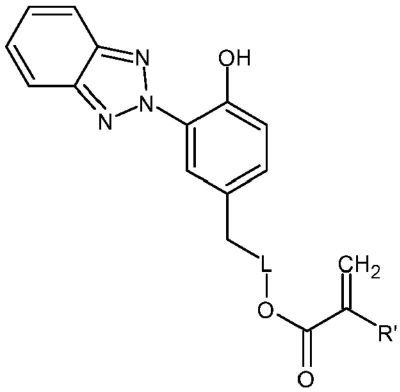

(III)

wherein

R' is a hydrogen atom or a $C_{1-4}$ alkyl group, and
L is a divalent linker;

for stabilizing a polymerized dental composition.

Preferably, the compound of formula (III) is a compound of the following formula (III-a):

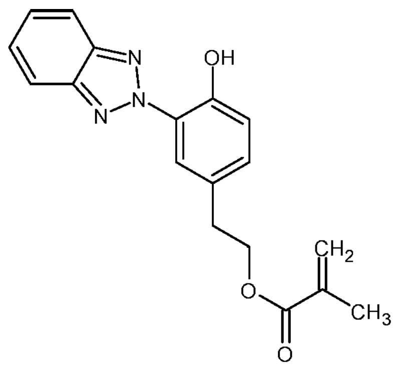

(III-a)

The present invention will now be further explained based on the following examples:
Isomers in the sense of this invention are stereoisomers, including enantiomers, diastereomers and constitutional isomers. Inhibitor in the sense of this invention is a polymerization inhibitor.

## Examples

The following abbreviations and terms are used in the current specification:

UDMA-Resin = Urethane dimethacrylate resin
DMABE = N,N-Dimethylamino ethyl benzoate
DPI = Dimethyl diphenyl iodonium hexafluorophosphate
EBPADMA = Ethoxylated bisphenol A dimethacrylate
BAABE = N,N'-diallyl-1,4- bisacrylamido-(2E)-but-2-ene
BADEP = N,N'-diethyl-1,3-bisacrylamido-propane
CQ = Camphorquinone
Spectrum TPH = Spectrum Total Performance Hybrid
BHT = Butylated hydroxytoluene
Paste = A dental composition comprising a filler
TEGDMA = Triethylenglycol dimethacrylate
BisEMA = [2-ethoxy-3-[4-[2-[4-[2-ethoxy-3-(2-methylprop-2-enoyloxy)propoxy]phenyl]propan-2-yl]phenoxy]propyl] 2-methylprop-2-enoate
BisGMA = Bisphenol A-glycidyl methacrylate
TCDDA = Tricyclodecane dimethanol diacrylate (Compound of formula (I))
TCDDMA = Tricyclodecane dimethanol dimethacrylate (Compound of formula (I))
UCDPMAA = a mixture of monomers, comprising urethane cyclohexyl diphenoxy propenyl methacryl acrylate (compound of formula (II))

### Reference Example 1: Preparation of Urethane Monomer (UCDPMAA)

A 500 mL flask was charged with 38.8 grams (0.200 mol) of 1,3-bis(isocyanatomethyl)cyclohexane under dry nitrogen flow and heated to about 60° C. under positive nitrogen pressure. To this reaction mixture, 3 drops of catalyst dibutyltin dilaurate were added. A mixture of 22.7 grams of 2-hydroxy-3-phenoxy propyl acrylate, 26.6 grams (0.204 mol) of 2-hydroxyethyl methacrylate, 11.5 grams (0.099 mol) of 2-hydroxyethyl acrylate and 0.10 grams of BHT as an inhibitor were added over a period of 70 minutes while the reaction temperature was maintained between 56° C. and 78° C. After about four hours stirring, the heat was turned off, and monomer was collected from the flask as viscous liquid and stored in a dry atmosphere.

### Example 1: Producing UV-stabilized pastes.

Six pastes were produced comprising 30 wt% liquid (UCDPMAA, TCDDMA, CQ, BHT, DMABE, with different concentration of 2-(2-Hydroxy-5-methylphenyl)benzotriazol or 2-[3-(2H-Benzotriazol-2-yl)-4- hydroxyphenyl]ethyl methacrylate) and 70 wt% silanized glass filler according to table 1. In the first step, the liquids were produced by stirring the suspension at 50 °C until all additives were completely dissolved. Next, the silanized glass filler was added and mixed

(Hauschild SpeedMixer) three times at 2000 rpm for 2 min and then two times for 1 min at reduced pressure at 200 mbar. After that, the pastes were stored at 60°C for 1 h and then mixed again at 2000 rpm for 1 min at 200 mbar.

**Example 2: Measuring the UV stability.**

To measure the UV-stability of the different pastes, the materials were filled into a round metal mold with a diameter of approximately 20 mm and a thickness of 1 mm. The materials were photopolymerized for 2 min on each side using a LicuLite light oven with UV-filter. After removal from the mold, one side of the composite plates was covered with a sheet of a paper and the other one was exposed to artificial sunlight light (83500 Lux, Atlas SunTest CPS+). After that, the color of both sides was measured via a Datacolor 800. For the measurement of L-, a-, and b-value, the plates are placed on a white tile with a drop of 1 wt% sodium dodecyl sulfate solution. The calculation of the delta E value (Figure 1) was carried out using Formula 1.

Formula 1: to calculate the delta E value for testing of the stability against UV light.

$$\Delta E = \sqrt{(L_a - L_b)^2 + (a_a - a_b)^2 + (b_a - b_b)^2}$$

With

$L_a$ Bright/dark value not exposed to UV-light
$L_b$ Bright/dark value exposed to UV-light
$a_a$ Red/green value not exposed to UV-light
$a_b$ Red/green value exposed to UV-light
$b_a$ Yellow/blue value not exposed to UV-light
$b_b$ Yellow/blue value exposed to UV-light

**Example 3: Measuring of leaching.**

To measure the leaching of 2-(2-Hydroxy-5-methylphenyl)benzotriazol and 2-[3-(2H-Benzotriazol-2-yl)-4-hydroxyphenyl]ethyl methacrylate), five separate plates of each paste of example 1 were produced. Therefore, the pastes were placed into a mold with a diameter of approximately 15 mm and a thickness of 1 mm. The materials were photopolymerized for 2 min on each side using a LicuLite light oven with UV-filter. After removal from the mold, the plaques were deburred and weighed. Afterwards, the five plates of each paste were placed in acetonitrile in a screw top jar and stored for 72 h at room temperature. Then, the solution was analyzed using HPLC (Agilent 1260 Infinity II, mobile phase: Eluent A: water + 0.1 % TFA eluent B: ACN + 0.1 TFA, Gradient 20% eluent B (5 min) → (55 min →) 100% Eluent B (5 min)) (Figure 2).

Table 1: Composition of the different pastes with Tinuvin-methacrylate* (2-[3-(2H-Benzotriazol-2-yl)-4-hydroxyphenyl]ethyl methacrylate)) or Tinuvin** (2-(2-Hydroxy-5-methylphenyl)benzotriazole) and comprising 30 wt% liquid and 70 wt% glass filler.

| Pa ste | UCDP MAA | UCDP MAA | TCDD MA | TCDD MA | C Q | CQ | DMA BE | DMA BE | B HT | BH T | Stabiliz er 1* | Stabiliz er 1* | Stabiliz er 2** | Stabiliz er 2** | Gla ss | Gla ss |
|---|---|---|---|---|---|---|---|---|---|---|---|---|---|---|---|---|
| | [g] | [wt%] | [g] | [wt%] | [m g] | [wt %] | [mg] | [wt% ] | [m g] | [wt %] | [g] | [wt%] | [g] | [wt%] | [g] | [wt %] |
| A | 200 | 19.98 | 0.86 | 8.56 | 7.5 | 0.0 8 | 23 | 0.23 | 15 | 0.1 5 | 0.10 | 100 | n.b. | n.b. | 7.0 0 | 70. 00 |
| B | 2.06 | 20.58 | 0.88 | 8.82 | 7.5 | 0.0 8 | 23 | 0.23 | 15 | 0.1 5 | 0.02 | 0.15 | n.b. | n.b. | 7.0 0 | 70. 00 |
| C | 2.06 | 20.64 | 0.88 | 8.85 | 7.5 | 0.0 8 | 23 | 0.22 | 15 | 0.1 5 | 0.01 | 0.06 | n.b. | n.b. | 7.0 0 | 70. 00 |
| D | 200 | 19.98 | 0.86 | 8.56 | 7.5 | 0.0 8 | 23 | 0.23 | 15 | 0.1 5 | n.b. | n.b. | 0.10 | 100 | 7.0 0 | 70. 00 |
| E | 2.06 | 20.58 | 0.88 | 8.82 | 7.5 | 0.0 8 | 23 | 0.23 | 15 | 0.1 5 | n.b. | n.b. | 0.02 | 0.15 | 7.0 0 | 70. 00 |
| F | 2.06 | 20.64 | 0.88 | 8.85 | 7.5 | 0.0 8 | 23 | 0.22 | 15 | 0.1 5 | n.b. | n.b. | 0.01 | 0.06 | 7.0 0 | 70. 00 |

**Example 4: Preparation of liquids 1-14 for dental formulations**

Fourteen mixtures comprising UCDPMAA, TCDDA, TCDDMA, BisGMA, TEGDMA, and UDMA-Resin in varying amounts were prepared each containing 0.25 wt% CQ, 0.75 wt% DMABE and 0.50 wt% BHT. For this purpose, the monomers are mixed first and stirred at 50°C until complete homogenization. Afterwards, the additives are added and the mixtures are further stirred in the dark at 50°C. Every 15 minutes, they are checked and the time is noted when all compounds are homogeneously mixed.

**Example 5: Preparation of dental compositions A, B, C and D with filler**

To each of the fourteen liquids 1-14 (30 wt%) was added 70 wt% silanized glass filler and the mixtures were mixed (Hauschild SpeedMixer) three times at 1400 rpm for 2 min and then 8 to 14 times at 1200 rpm for 1 min at reduced pressure at 50 mbar.

**Example 6: Measurement of refractive index**

The refractive index of the liquid is determined via the angle of total reflection at $\lambda$ = 589 nm and 20°C on an Abbemat device (Figure 3).

**Example 7: Measurement of viscosity**

For the measurement of viscosity, the liquid is loaded on a Kinexus rheometer at 23°C and the sample is equilibrated for 30 s. The measurement is done at 1 Pa shear stress for low viscosity fluids, 10 Pa for medium viscosity fluids (~1.5 to ~50 Pa s) and 20 Pa for high viscosity fluids with a recovery time of 5 s.

**Example 8: Measurement of Flexural strength and E-modulus**

The material is filled into a metal mold of approximately 2x2x30 mm which is subsequently covered with foil and manually pressed. The material is photopolymerized for 2 min on each side using a LicuLite light oven with UV-filter. After removal from the mold, excess of material is removed by wet sanding with grit paper 1000. The specimens are conditioned in water at 37°C before they are subjected to a three-point-bending test at a Zwick universal testing machine. The span width is 20 mm, the feeding speed is 1 mm/min. For the determination of the E-modulus, the linear elastic region is used.

**Example 9: Measurement of opacity**

The material is filled into a round mold of 30 mm diameter and 1 mm thickness between two sheets of foil and manually pressed. It is photopolymerized for 2 min on each side using a LicuLite light oven with UV-filter. The color measurement is done at a Datacolor 800. For the measurement of L, a, and b-value, the plates are placed on a white tile with a drop of 1 wt% sodium dodecyl sulfate solution. The result is the average of the measurement at 5 points. The opacity value is then obtained by an additional measurement in the same fashion on a black tile (Figure 3).

**Table 2: Composition of liquid formulations.**

| Nr . | BisGMA | | UCDPMAA | | TCDDA | | TEGDMA | | UDMA-Resin | | TCDDMA | |
|---|---|---|---|---|---|---|---|---|---|---|---|---|
| | wt% | g | wt% | g | wt% | g | wt% | g | wt% | g | wt% | g |
| 1 | | | 98.5 0 | 11.8 2 | | | | | | | | |
| 2 | | | | | 98.5 0 | 11.8 2 | | | | | | |
| 3 | | | 68.9 5 | 8.27 | 29.5 5 | 3.55 | | | | | | |
| 4 | | | 29.5 5 | 3.55 | 68.9 5 | 8.27 | | | | | | |
| 5 | | | 68.9 5 | 8.27 | | | 29.5 5 | 3.5 5 | | | | |
| 6 | | | 29.5 5 | 3.55 | | | 68.9 5 | 8.2 7 | | | | |
| 7 | 68.9 5 | 8.2 7 | | | | | 29.5 5 | 3.5 5 | | | | |
| 8 | 29.5 5 | 3.5 5 | | | | | 68.9 5 | 8.2 7 | | | | |
| 9 | 68.9 5 | 8.2 7 | | | 29.5 5 | 3.55 | | | | | | |

(continued)

| Nr . | BisGMA | | UCDPMAA | | TCDDA | | TEGDMA | | UDMA-Resin | | TCDDMA | |
|---|---|---|---|---|---|---|---|---|---|---|---|---|
| | wt% | g | wt% | g | wt% | g | wt% | g | wt% | g | wt% | g |
| 10 | 29.5 5 | 3.5 5 | | | 68.9 5 | 8.27 | | | | | | |
| 11 | | | 29.5 5 | 3.55 | | | | | | | 68.9 5 | 8.2 7 |
| 12 | | | 68.9 5 | 8.27 | | | | | | | 29.5 5 | 3.5 5 |
| 13 | | | 32.8 3 | 3.94 | 32.8 3 | 3.94 | | | 32.8 3 | 3.94 | | |
| 14 | | | 32.8 3 | 3.94 | | | 32.8 3 | 3.9 4 | 32.8 3 | 3.94 | | |

| Nr. | CQ | | DMABE | | BHT | |
|---|---|---|---|---|---|---|
| | wt% | g | wt% | g | wt% | g |
| 1 | 0.25 | 0.03 | 0.75 | 0.09 | 0.50 | 0.06 |
| 2 | 0.25 | 0.03 | 0.75 | 0.09 | 0.50 | 0.06 |
| 3 | 0.25 | 0.03 | 0.75 | 0.09 | 0.50 | 0.06 |
| 4 | 0.25 | 0.03 | 0.75 | 0.09 | 0.50 | 0.06 |
| 5 | 0.25 | 0.03 | 0.75 | 0.09 | 0.50 | 0.06 |
| 6 | 0.25 | 0.03 | 0.75 | 0.09 | 0.50 | 0.06 |
| 7 | 0.25 | 0.03 | 0.75 | 0.09 | 0.50 | 0.06 |
| 8 | 0.25 | 0.03 | 0.75 | 0.09 | 0.50 | 0.06 |
| 9 | 0.25 | 0.03 | 0.75 | 0.09 | 0.50 | 0.06 |
| 10 | 0.25 | 0.03 | 0.75 | 0.09 | 0.50 | 0.06 |
| 11 | 0.25 | 0.03 | 0.75 | 0.09 | 0.50 | 0.06 |
| 12 | 0.25 | 0.03 | 0.75 | 0.09 | 0.50 | 0.06 |
| 13 | 0.25 | 0.03 | 0.75 | 0.09 | 0.50 | 0.06 |
| 14 | 0.25 | 0.03 | 0.75 | 0.09 | 0.50 | 0.06 |

**Table 3: Composition of dental compositions 1-14 from liquids 1-14 of Table 2.**

| **Liquid number 1-14 [wt%]** | **Liquid number 1-14 [g]** | **Glass [wt%]** | **Glass [g]** |
|---|---|---|---|
| 30 | 3 | 70 | 7 |

**Table 4: Characteristics of the liquids and dental compositions of Table 2 and Table 3.**

| **Nr.** | **Liquid properties** | | | **Paste properties** | | |
|---|---|---|---|---|---|---|
| | Refractive index | Viscosity [Pa s] | Time to dissolve additives [min] | Opacity [%] | Flexural strength [MPa] | E-modulus [MPa] |
| 1 | 1.5096 | 626 | 60 | 13.3 | 158 | 9550 |
| 2 | 1.5050 | 0.187 | 15 | 9.8 | 110 | 7410 |
| 3 | 1.5081 | 16.4 | 15 | 10.7 | 140 | 8940 |
| 4 | 1.5063 | 0.811 | 15 | 9.8 | 126 | 8860 |
| 5 | 1.4945 | 1.37 | 15 | 14.9 | 158 | 9010 |
| 6 | 1.4752 | 0.0382 | 15 | 23.2 | 118 | 7280 |
| 7 | 1.5224 | 2.13 | 15 | 21.5 | 138 | 8110 |
| 8 | 1.4869 | 0.0470 | 15 | 11.5 | 112 | 5020 |

(continued)

| Nr. | Liquid properties | | | Paste properties | | |
|---|---|---|---|---|---|---|
| | Refractive index | Viscosity [Pa s] | Time to dissolve additives [min] | Opacity [%] | Flexural strength [MPa] | E-modulus [MPa] |
| 9 | 1.5358 | 31.5 | 15 | 29.9 | 119 | 8750 |
| 10 | 1.5179 | 1.05 | 15 | 13.3 | 122 | 8330 |
| 11 | 1.5034 | 0.816 | 15 | 10.6 | 122 | 8630 |
| 12 | 1.5066 | 19.1 | 30 | 11.0 | 134 | 8910 |
| 13 | 1.4970 | 5.44 | 30 | 17.6 | 132 | 8610 |
| 14 | 1.4875 | 0.516 | 15 | 25.6 | 132 | 7620 |

All the above described characteristics and their units are summarized in the following table:

**Table 5: list of abbreviations and terms.**

| Abbreviation/Term | Characteristic/definition | Unit |
|---|---|---|
| Refractive index | Speed of light in vacuum devided by speed of light in matrix | -- |
| Viscosity | Resistance to deformation of a fluid / thickness | Pa s |
| FS | Flexural Strength | MPa |
| E-Modulus | Modulus of elasticity under flexural stress | MPa |
| Opacity | Light impenetrability of the material | % |

## Claims

**1.** A polymerizable dental composition comprising

(a) 10 to 99 percent by weight based on the total weight of the dental composition of a homogenous mixture of polymerizable monomers, which comprises

(a1) 5 to 99.5 percent by weight based on the total weight of the mixture of a first polymerizable component comprising one or more thinner compounds of the following formula (I):

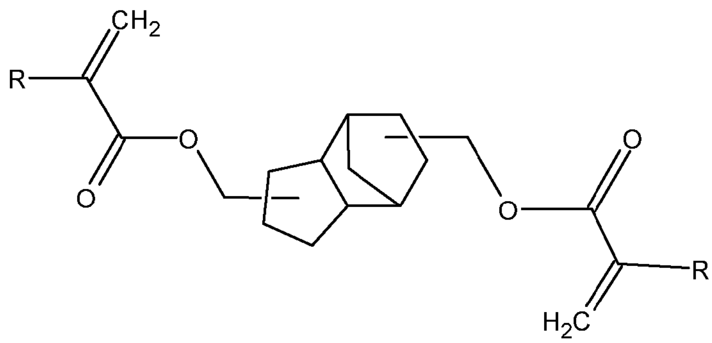

(I)

wherein the R, which may be the same or different, independently from each other represent a hydrogen atom or a $C_{1-4}$ alkyl group which may be substituted by one or more fluorine atoms; and
(a2) 0.5 to 90 percent by weight based on the total weight of the mixture of one or more performer compounds of the following formula (II):

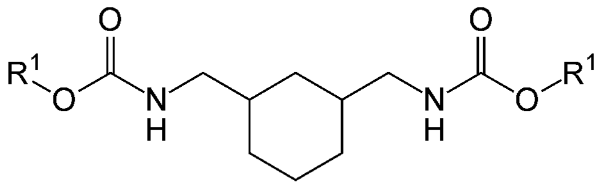

(II)

wherein the $R^1$ which may be the same or different, independently from each other is selected from a group of the following formulas (II-a) or (II-b):

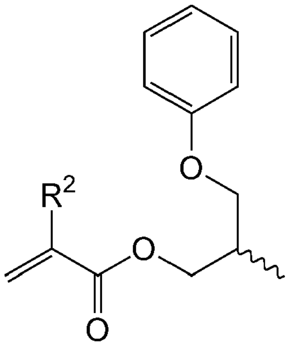

(II-a)

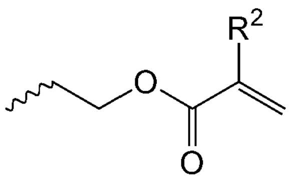

(II-b)

wherein $R^2$ represents a hydrogen atom or a $C_{1-4}$ alkyl group which may be substituted by one or more fluorine atoms; and

(a3) optionally a third polymerizable component;

(b) a polymerization initiator system, and

(c) 10 to 90 percent by weight of a particulate filler.

**2.** The polymerizable dental composition according to claim 1, wherein the third polymerizable component (a3) is contained in an amount of 0.001 to 90 percent by weight, preferably 0.01 to 70 percent by weight, more preferably 0.01 to 60 percent by weight, especially 0.01 to 40 percent by weight based on the total weight of the mixture, of a third polymerizable component.

**3.** The polymerizable dental composition according to claim 1 or 2, which further comprises 0.001 to 10 percent by weight, preferably 0.01 to 7 percent by weight, particularly 0.05 to 6 percent by weight, especially 0.05 to 5 percent by weight based on the total weight of the dental composition of at least one UV-stabilizer, preferably wherein the UV-stabilizer is a compound of the following formula (III):

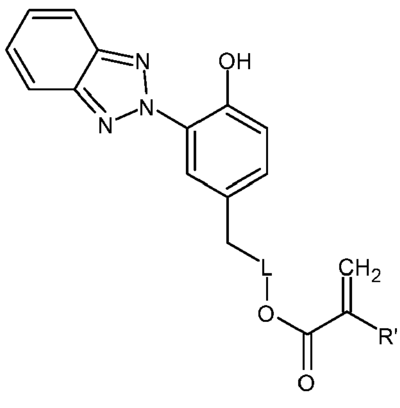

(III)

wherein

R' is a hydrogen atom or a $C_{1-4}$ alkyl group, and
L is a single bond or a divalent linker.

**4.** The polymerizable dental composition according to any one of the preceding claims, which further comprises 0.0001 to 10 percent by weight, preferably 0.001 to 7 percent by weight, particularly 0.005 to 6 percent by weight, especially 0.005 to 5 percent by weight based on the total weight of the dental composition of at least one polymerization inhibitor.

**5.** The polymerizable dental composition according to any one of the preceding claims, which further comprises 0.0001 to 1 percent by weight based on the total weight of the dental composition of at least one pigment.

**6.** The polymerizable dental composition according to any one of the preceding claims wherein the particulate filler comprises a glass.

**7.** The polymerizable dental composition according to any one of the preceding claims wherein the first polymerizable component comprises at least two compounds of formula (I), preferably wherein the two or more compounds are enantiomers, diastereomers and constitutional isomers; and/or wherein the one or more compounds of formula (I) comprise or consist of compounds of the following formula (I-a) and/or (I-b):

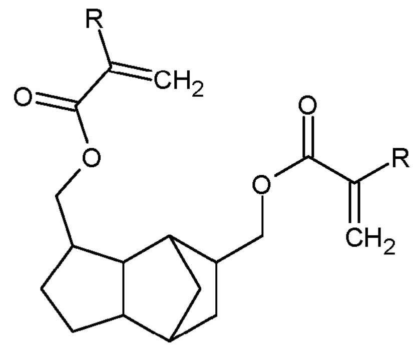

(I-a)

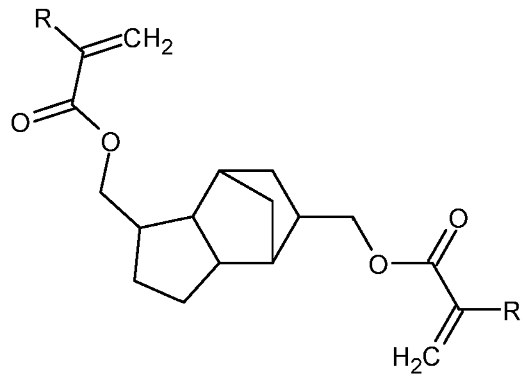

(I-b)

wherein the R, which may be the same or different, independently are as defined in claim 1; and/or

wherein R represents a hydrogen atom; and/or
wherein the ratio of the refractive index of the component (a1) and the refractive index of component (a2) $n_{(a1)}$ / $n_{(a2)}$ is in the range of 0.95 to 1.05; and/or
wherein the third polymerizable component comprises one or more polymerizable monomers selected from bisphenol A-glycidyl methacrylate (BisGMA), urethane dimethacrylate (UDMA), and ethoxylated bisphenol A dimethacrylate (BisEMA), polycarbonate urethane dimethacrylate (PCUDMA).

**8.** The polymerizable dental composition according to any one of claims 3 to 7,

wherein in the compound of formula (III), R' is a hydrogen atom or a methyl group; and/or
wherein L is an alkylene group or a polyoxyalkylene group; and/or
which contains 0.001 to 5 percent by weight based on the total amount of the total weight of the dental composition a compound of formula (III); and/or
wherein the compound of formula (III) is a compound of the following formula (III-a):

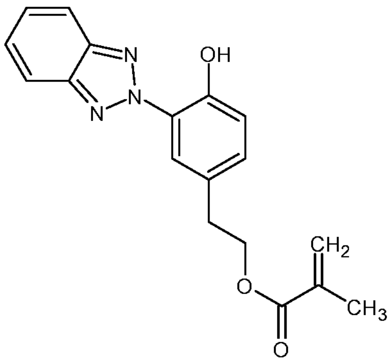

(III-a)

and/or
which comprises the particulate filler in an amount of from 40 to 90 percent by weight based on the total weight of the composition.

**9.** A use of a compound of the following formula (III):

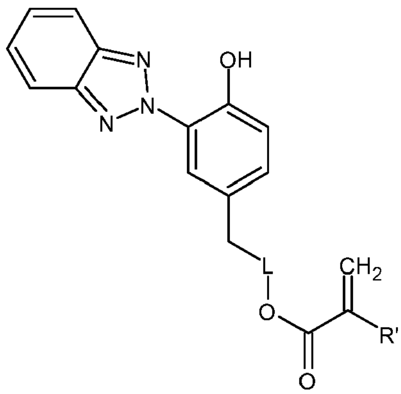

(III)

wherein

R' is a hydrogen atom or a $C_{1-4}$ alkyl group, and
L is a divalent linker;

for stabilizing a polymerized dental composition.

**10.** The use according to claim 9, wherein the compound of formula (III) is a compound of the following formula (III-a):

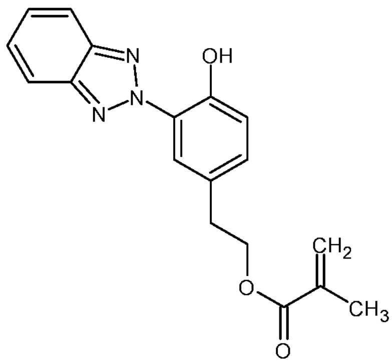

(III-a)

**Fig. 1**

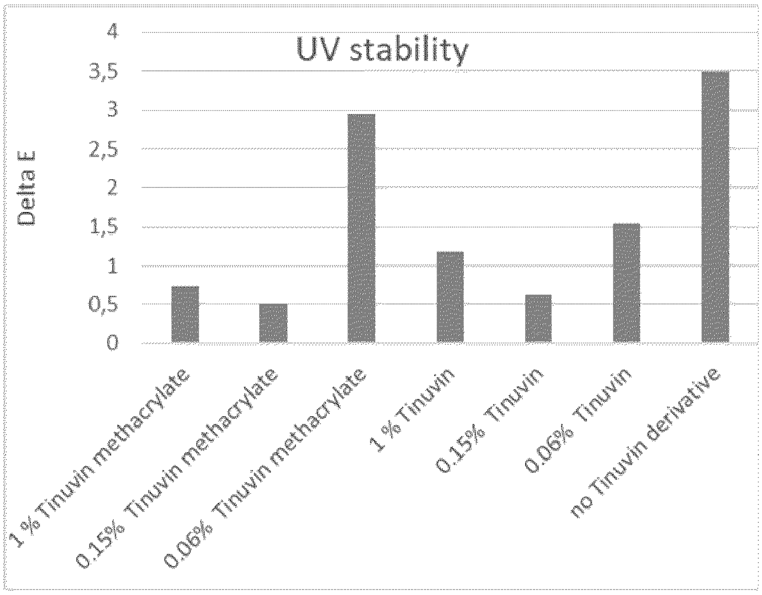
UV stability
Delta E
4
3,5
3
2,5
2
1,5
1
0,5
0
1 % Tinuvin methacrylate
0.15% Tinuvin methacrylate
0.06% Tinuvin methacrylate
1 % Tinuvin
0.15% Tinuvin
0.06% Tinuvin
no Tinuvin derivative

**Fig. 2**

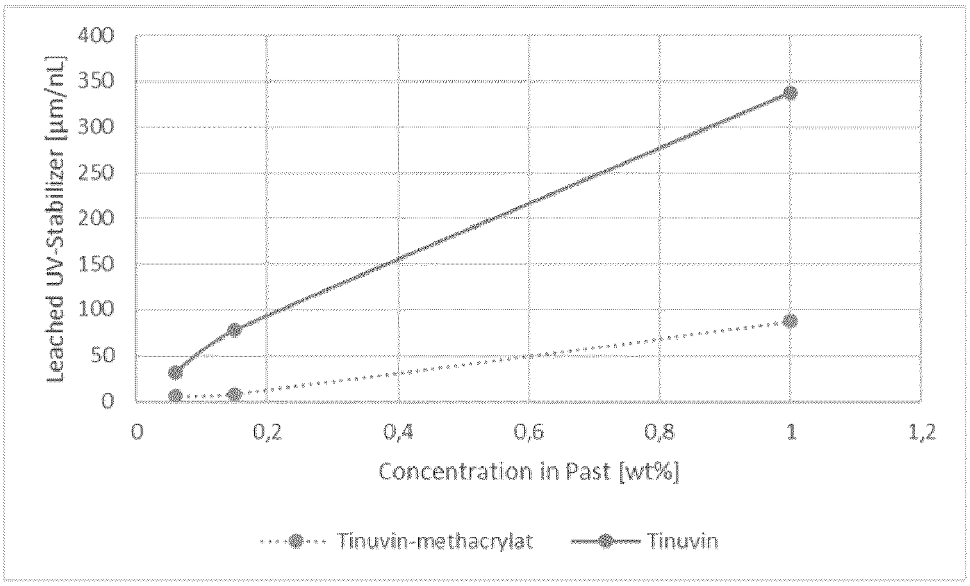
Leached UV-Stabilizer [µm/nL]
400
350
300
250
200
150
100
50
0
0
0,2
0,4
0,6
0,8
1
1,2
Concentration in Past [wt%]
Tinuvin-methacrylat
Tinuvin

**Fig. 3**

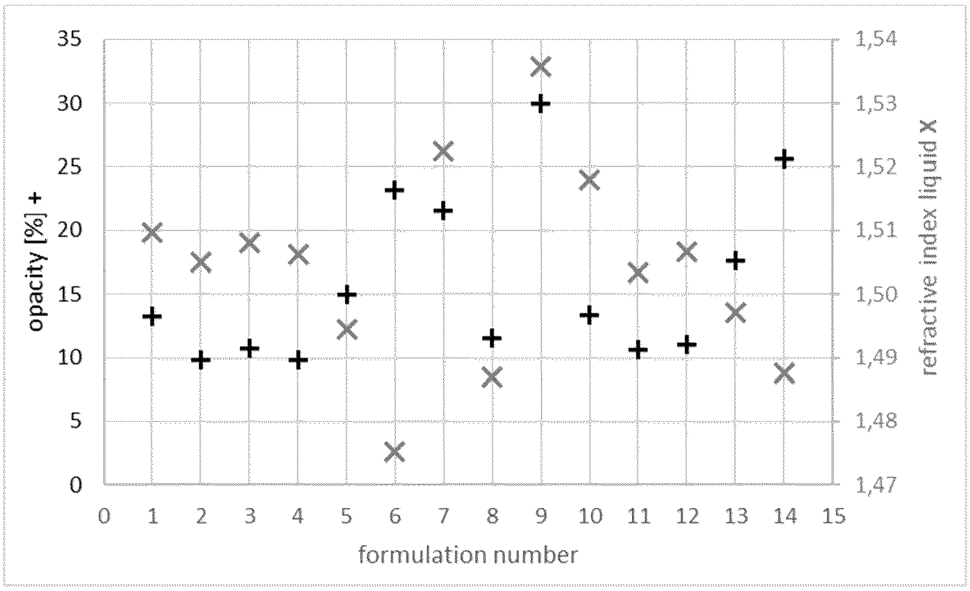

35
30
25
20
15
10
5
0
1,54
1,53
1,52
1,51
1,50
1,49
1,48
1,47
0 1 2 3 4 5 6 7 8 9 10 11 12 13 14 15
opacity [%] +
refractive index liquid x
formulation number

Europäisches Patentamt
European Patent Office
Office européen des brevets

# EUROPEAN SEARCH REPORT

Application Number
EP 24 21 0551

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2005 255681 A (IVOCLAR VIVADENT AG) 22 September 2005 (2005-09-22) * structure on page 13; claims 1-10 * ----- | 9,10 | INV. A61K6/16 A61K6/62 A61K6/77 A61K6/836 A61K6/887 |
| A | WO 2020/109564 A1 (UNIV CATHOLIQUE LOUVAIN [BE]) 4 June 2020 (2020-06-04) * claims 1-6; examples 1-4 * ----- | 1-8 | |
| A | US 2014/131908 A1 (SUN BENJAMIN JIEMIN [US] ET AL) 15 May 2014 (2014-05-15) * examples 20, 22, 23 * ----- | 1-8 | |
| A | US 2018/000570 A1 (SUN BENJAMIN JIEMIN [US] ET AL) 4 January 2018 (2018-01-04) * example 4 * ----- | 1-8 | |
| A | US 2017/360534 A1 (SUN BENJAMIN J [US] ET AL) 21 December 2017 (2017-12-21) * examples 11-13 * ----- | 1-8 | TECHNICAL FIELDS SEARCHED (IPC) |
| A | US 2012/296061 A1 (NARUSE HIROSHI [JP] ET AL) 22 November 2012 (2012-11-22) * claims 1, 11-15; examples 100-112 * ----- | 1-8 | A61K |
| A | US 2012/296003 A1 (NARUSE HIROSHI [JP] ET AL) 22 November 2012 (2012-11-22) * claims 1, 11-14; examples 100-112 * ----- | 1-8 | |

The present search report has been drawn up for all claims

1

| Place of search | Date of completion of the search | Examiner |
|---|---|---|
| The Hague | 24 February 2025 | Pelras, Théophile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 21 0551

This annex lists the patent family members relating to the patent documents cited in the above-mentioned European search report. The members are as contained in the European Patent Office EDP file on
The European Patent Office is in no way liable for these particulars which are merely given for the purpose of information.

24-02-2025

| Patent document cited in search report | Publication date | Patent family member(s) | Publication date |
|---|---|---|---|
| JP 2005255681 A | 22-09-2005 | AT E410452 T1 | 15-10-2008 |
| | | DE 102004011497 A1 | 06-10-2005 |
| | | EP 1574524 A1 | 14-09-2005 |
| | | JP 4782444 B2 | 28-09-2011 |
| | | JP 2005255681 A | 22-09-2005 |
| | | JP 2011173920 A | 08-09-2011 |
| | | US 2005203199 A1 | 15-09-2005 |
| WO 2020109564 A1 | 04-06-2020 | BE 1026813 A1 | 23-06-2020 |
| | | WO 2020109564 A1 | 04-06-2020 |
| US 2014131908 A1 | 15-05-2014 | BR 112015010983 A2 | 11-07-2017 |
| | | CA 2889331 A1 | 22-05-2014 |
| | | CA 3081240 A1 | 22-05-2014 |
| | | CN 104853693 A | 19-08-2015 |
| | | EP 2919705 A1 | 23-09-2015 |
| | | ES 2879602 T3 | 22-11-2021 |
| | | HK 1212581 A1 | 17-06-2016 |
| | | JP 6197043 B2 | 13-09-2017 |
| | | JP 2016505525 A | 25-02-2016 |
| | | RU 2015122752 A | 10-01-2017 |
| | | US 2014131908 A1 | 15-05-2014 |
| | | WO 2014078537 A1 | 22-05-2014 |
| US 2018000570 A1 | 04-01-2018 | CA 3029491 A1 | 04-01-2018 |
| | | EP 3478220 A1 | 08-05-2019 |
| | | ES 2834622 T3 | 18-06-2021 |
| | | JP 7010859 B2 | 26-01-2022 |
| | | JP 2019520912 A | 25-07-2019 |
| | | US 2018000570 A1 | 04-01-2018 |
| | | WO 2018005900 A1 | 04-01-2018 |
| US 2017360534 A1 | 21-12-2017 | CA 3026166 A1 | 28-12-2017 |
| | | EP 3472218 A1 | 24-04-2019 |
| | | ES 2957886 T3 | 29-01-2024 |
| | | JP 7036810 B2 | 15-03-2022 |
| | | JP 2019521188 A | 25-07-2019 |
| | | US 2017360534 A1 | 21-12-2017 |
| | | US 2019053883 A1 | 21-02-2019 |
| | | WO 2017223084 A1 | 28-12-2017 |
| US 2012296061 A1 | 22-11-2012 | CN 103492446 A | 01-01-2014 |
| | | EP 2711381 A1 | 26-03-2014 |
| | | JP 5819415 B2 | 24-11-2015 |
| | | JP WO2012157568 A1 | 31-07-2014 |
| | | US 2012296061 A1 | 22-11-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 24 21 0551

This annex lists the patent family members relating to the patent documents cited in the above-mentioned European search report. The members are as contained in the European Patent Office EDP file on
The European Patent Office is in no way liable for these particulars which are merely given for the purpose of information.

24-02-2025

| Patent document cited in search report | Publication date | Patent family member(s) | Publication date |
|---|---|---|---|
| | | WO 2012157568 A1 | 22-11-2012 |
| US 2012296003 A1 | 22-11-2012 | CN 103491928 A | 01-01-2014 |
| | | EP 2710994 A1 | 26-03-2014 |
| | | JP 5931057 B2 | 08-06-2016 |
| | | JP 2016094482 A | 26-05-2016 |
| | | JP WO2012157566 A1 | 31-07-2014 |
| | | US 2012296003 A1 | 22-11-2012 |
| | | WO 2012157566 A1 | 22-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10849724 B
- US 4298738 A
- US 4324744 A
- US 4385109 A
- EP 0173567 A
- US 5501727 A
- US 5154762 A
- EP 2604247 A